# EUROPEAN PATENT SPECIFICATION

(11) **EP 1 573 018 B1**
(45) Date of publication and mention of the grant of the patent: **15.04.2009**
(21) Application number: 02781684.2
(22) Date of filing: 16.12.2002
(51) Int. Cl.: C12P 41/00, C12P 11/00

(54) **CHEMOENZYMATIC PROCESS FOR STEREOSELECTIVE PREPARATION OF R- AND S-ENANTIOMERS OF 2-HYDROXY-3-(2-THIENYL) PROPANENITRILE**
CHEMOENZYMATISCHES VERFAHREN ZUR STEREOSELEKTIVEN HERSTELLUNG DER R- UND S-ENANTIOMERE VON 2-HYDROXY-3-(2-THIENYL) PROPANNITRIL
PROCEDE CHIMIO-ENZYMATIQUE DE PREPARATION STEREOSELECTIVE D'ENANTIOMERES R ET S DE 2-HYDROXY-3-(2-THIENYL) PROPANENITRILE

(43) Date of publication of application: 14.09.2005
(73) Proprietor: Council of Scientific and Industrial Research, New Delhi 100 001 (IN)
(72) Inventor: KAMAL, A. Indian Institute of Chemical Technology, Andhra Pradesh (IN); GOLLAPALLI, B. R. K. Indian Inst. of Chem. Techn., Andhra Pradesh (IN); RONDLA, R. Indian Institute of Chemical Technology, Andhra Pradesh (IN); MADDAMSETTY, V. R. Indian Inst. of Chem. Techn., Andhra Pradesh (IN)
(74) Representative: Smyth, Gyles Darren
(86) International application number: PCT/IB2002/005508
(87) International publication number: WO 2004/055194

(56) References cited:
- WO-A-02/057475
- SAKAI T ET AL: "Lipase-Catalyzed Efficient Kinetic Resolution of 3-Hydroxy-3-(pentafluorophenyl)propionitri le" TETRAHEDRON LETTERS, ELSEVIER SCIENCE PUBLISHERS, AMSTERDAM, NL, vol. 38, no. 11, 17 March 1997 (1997-03-17), pages 1987-1990, XP004055834 ISSN: 0040-4039
- ITOH TOSHIYUKI ET AL: "Enhanced enantioselectivity of an enzymatic reaction by the sulfur functional group. A simple preparation of optically active.beta.-hydrox nitriles using a lipase" JOURNAL OF ORGANIC CHEMISTRY, AMERICAN CHEMICAL SOCIETY. EASTON, US, vol. 56, no. 4, 1991, pages 1521-1524, XP002179192 ISSN: 0022-3263

## Description

### Field of the invention

The present invention relates to a chemoenzymatic process for stereoselective preparation of both *R* and *S* enantiomers of 3-hydroxy-3-(2-thienyl) propanenitrile **(1).**

This invention particularly relates to a chemoenzymatic process for the stereoselective preparation of both the enantiomers of 3-hydroxy-3-(2-thienyl) propanenitrile **(2)** a key intermediate for the synthesis of antipsychic drug duloxetine.

### Background of the invention

Duloxetine and related class of compounds like fluoxetine, tomoxetine etc., are important for treating psychiatric disorders. Fluoxetine is a selective inhibitor of serotonin in serotonergic neurons, tomoxetine and nisoxetine are selective inhibitors of norepinephrine in noradrenergic neurons while duloxetine is a dual inhibitor of serotonin and norepinephrine reuptake and thus have a better pharmacological profile as an antidepressant drug (EP 273658, 1988*;* Chem. Abstr., 1988, 109, 170224n; Life Sci. 1988, 43, 2049).

Serotonin and norepinephrine neuro transmitters are intimately involved in a number of physiological and behavioral processes, suggesting that duloxetine (ability to produce robust increase of extra cellular serotonin and norepinephrine levels) is not only a highly efficient antidepressant agent for treating psychiatric disorders but also can be used for treating other symptoms like alcoholism, urinary incontinence, fatigue, stroke, intestinal cystitis, obsessive compulsive disorder, panic disorder, hyperactivity disorder,¹¹ sleep disorder, sexual dysfunction etc.

Duloxetine having one chiral center can exist in two isomeric forms. In view of the different pharmacological activities displayed by individual enantiomers, differences in metabolic behaviour and importance to provide enantiomerically pure forms as drugs, the preparation of this drug in enantiomerically pure form is highly desirable.

Duloxetine has the basic structural skeleton of 3-amino-propanol and retrosynthetic synthetic strategy reveals that enantiomerically pure 3-hydroxy-3-(2-thienyl) propanenitrile (β-hydroxy nitrile) **(1)** should be an excellent chiral building block for the synthesis of the target molecule. In the literature, there are only few reports for the synthesis of duloxetine in optically pure form, one by employing complexed-LAH for asymmetric reduction of the Mannich base *(*Tetrahedron Lett. 1990, 31, 7101) and other by lipase mediated resolution of 3-chloro-1-(2-thienyl)-1-propanol *(*Chirality 2000,12,26). The literature discloses also a lipase-catalyzed efficient kinetic resolution of 3-hydroxy-3-(pentafluorophenyl)propionitrile (Tetrahedron Letters 1997, 38, 1987)

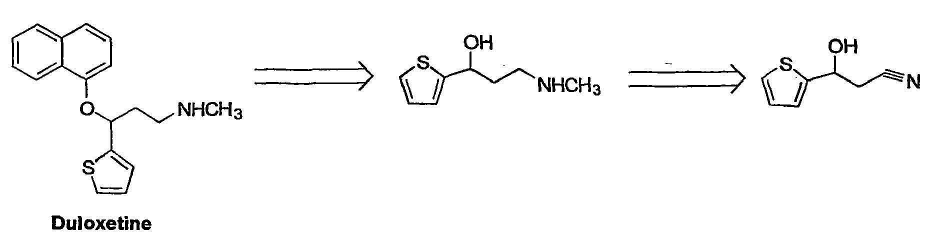

### Object of the invention

The main object of the invention is to provide a chemoenzymatic process for the stereoselective preparation of both the enantiomers of 3-hydroxy-3-(2-thienyl) propanenitrile (**1**) which are optically pure intermediates for the synthesis of both enantiomers of duloxetine in high enantiomeric excess.

In the drawings accompanying this specification represents enzymatic resolution of 3-hydroxy-3-(2-thienyl) propanenitrile via lipase mediated transesterification of 3-hydroxy-3-(2-thienyl) propanenitrile **(1)** and lipase mediated hydrolysis of 3-acetyloxy-3-(2-thienyl) propanenitrile **(2)** to afford optically pure hydroxy nitrile and its corresponding acetate. **Summary of the invention**

Accordingly the present invention provides a chemoenzymatic process for the stereoselective preparation of both *R* and *S* enantiomers of 3-hydroxy-3-(2-thienyl) propanenitrile (**1),** which comprises (i) reacting the cyanohydrin (**1**) with an acetylating agent in the presence of lipase and then separating the (*R*)-acetate and (*S*)-alcohol obtained ii) enzymatically hydrolysing 3-acetyloxy-3-(2-thienyl)propanenitrile (**2**) in a phosphate buffer solution to provide (*S*)-acetate and (*R*)-alcohol.

In one embodiment of the invention, recycling is not required for the products obtained by kinetic resolution since the enantiomeric excess is >99%.

In another embodiment of the invention, the acetylating agent is selected from the group consisting of vinyl acetate or isopropenyl acetate.

In yet another embodiment of the invention, the acetylation of the cyanohydrin is done in the presence of a lipase in a solvent.

In a further embodiment of the invention, the solvent is selected from the group consisting of diisopropyl ether, toluene, hexane, dioxane, chloroform, t-butylmethyl ether, diethyl ether, tetrahydrofuran, acetonitrile and acetone.

In yet another embodiment of the invention, the lipase used is selected from the group consisting of *Pseudomonas cepacia* lipase immobilized on modified ceramic particles (PS-C), *Pseudomonas cepacia* lipase immobilized on diatomite (PS-D), *Psedomonas cepacia* (PS), Porcine pancreas lipase (PPL), *Candida cylindracea* lipase (CCL), *Candida rugosa* lipase (CRL) and lipase immobilized *from Mucor miehei* (Lipozyme).

### Detailed description of the invention

The present invention provides an enzymatic process for the stereoselective preparation of both the enantiomers of 3-hydroxy-3-(2-thienyl) propanenitrile (**1**), a key intermediate for the synthesis of both enantiomers of duloxetine which comprises of reacting the cyanohydrin with acetylating agent and hydrolysis of acetyloxy nitrile in the presence of lipase followed by separation of acetate and alcohol obtained employing column chromatography. The 3-hydroxy-3-(2-thienyl) propanenitrile (**1**) was selectively acetylated with vinyl acetate, isopropenyl acetate in the presence of lipases while 3-acetyloxy-3-(2-thienyl) propanenitrile (**2**) was selectively hydrolyzed in phosphate buffer in the presence of the lipases.

The alcohol and the ester formed in the kinetic resolution were separated by column chromatography. The enantiomeric purities of the compounds were determined by HPLC employing a chiral column (OJ-H). Absolute configurations were preliminarily presumed to be *R* for acetate and *S* for alcohol (in the transesterification reaction) by the empirical rule for the enantiopreference of the lipase and then later confirmed from the optical rotation values of their corresponding aminoalcohols (Chirality 2000,12, 26).

The process of the present invention is illustrated below:
1. Racemic 3-hydroxy-3-(2-thienyl)propanenitrile (**1**) was acetylated enzymatically employing different acetylating agents and various lipases in different solvents
2. Acetylating agents such as vinyl acetate and isopropenyl acetate were used for acetylation.
3. Different lipases like *Pseudomonas cepacia* lipase immobilized on modified ceramic particals (PS-C), *Pseudomonas cepacia* lipase immobilized on diatomite (PS-D), *Psedomonas cepacia* (PS), porcine pancreas lipase (PPL), *Candida cylindracea* lipase (CCL), *Candida rugosa* lipase (CRL), lipase immobilized *from Mucor miehei* (Lipozyme) were screened for the enzymatic transesterification process.
4. Various solvents like diisopropyl ether, toluene, hexane, dioxane, chloroform, t-butylmethyl ether, diethyl ether, tetrahydrofuran, acetonitrile and acetone were employed.
5. Racemic 3-acetyloxy-3-(2-thienyl) propanenitrile (**2**) was prepared by reaction of the hydroxy nitrile with acetic anhydride in the presence of pyridine.
6. Racemic 3-acetyloxy-3-(2-thienyl) propanenitrile (**2**) was hydrolyzed enzymatically employing various lipases in phosphate buffer solution.
7. In the presence of lipases (*R*)-3-hydroxy-3-(2-thienyl)propanenitrile was selectively acetylated, and (*R*)-3-acetyloxy-3-(2-thienyl)propanenitrile was selectively hydrolyzed and the thus obtained acetates and alcohols were separated by column chromatography.
8. The enantiomeric excess was determined by HPLC employing chiral column. The reaction mechanism is given below:

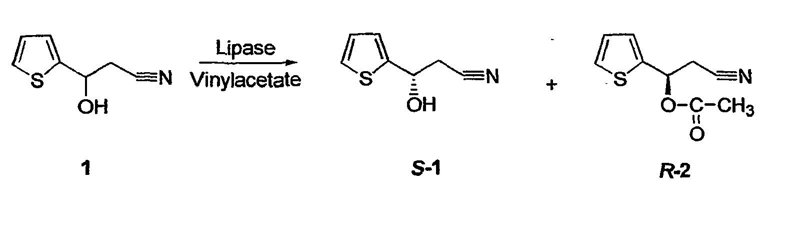

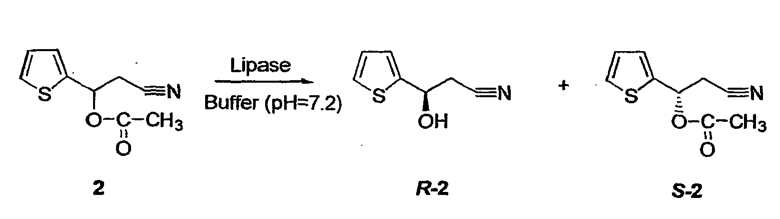

The following examples are given by way of illustration and they should not be construed to limit the scope of the present invention.

### Example 1

Enzymatic Transesterification of 3-hydroxy-3-(2-thienyl) propanenitrile (**1**) **:** To a solution of 3-hydroxy-3-(2-thienyl) propanenitrile (**1**) (5 mmol) dissolved in diisopropyl ether (100 mL), *Pseudomonas cepacia* lipase immobilized on diatomite (PS-D) (500 mg) and vinyl acetate (25 mmol) were added successively and incubated at 25°C in an orbital shaker. After 50% completion of the reaction (14 h) as indicated by HPLC, the reaction mixture was filtered and solvent was evaporated under reduced pressure. The residue was subjected to column chromatography to separate the acetate formed and the unreacted alcohol. The optical purity of these compounds were determined by HPLC. (S)-3-hydroxy-3-(2-thienyl) propanenitrile (***S*-1**) 42% yield, ee >99%; [α]³⁰_{D} = -33.5 (c 1, CHCl₃); IR (Neat) 3475, 3075, 2878, 2251, 1105, 698 cm⁻¹; ¹H NMR (200 MHz, CDCl₃) δ 2.82 (dd, 2H, J = 3.0 Hz, J = 6.6 Hz), 3.0 (s, 1H), 5.25 (t, 1H, J = 5.5 Hz), 6.98 (t, 1H, J = 4.9 Hz), 7.05 (d, 1H, J = 3.7 Hz), 7.28 (d, 1H, J= 4.9 Hz); ¹³C NMR (75 MHz) δ 28.1, 66.0, 117.0, 124.6, 125.6, 127.0, 144.4; Mass (EI) 153, 127, 113, 85.

(R)-3-acetyloxy-3-(2-thienyl) propanenitrile (***R*-2**) 43% yield, ee >99%, [α]³⁰_{D} = +83.6 (c 1, CHCl₃); IR (Neat) 3114, 2910, 2800, 2243,1734, 1216 cm⁻¹; ¹H NMR (200 MHz, CDCl₃) δ 2.10 (s, 3H), 2.94 (d, 2H, J = 6.7 Hz), 6.20 (t, 1H, J = 6.7 Hz), 6.97 (t, 1H, J = 3.8 Hz), 7.1 (d, 1H, 3.0 Hz), 7.3 (d, 1H, J = 5.9 Hz); Mass (EI) 195,154,137,114,85,43.

### Example 2

Hydrolysis of 3-acetyloxy-3-(2-thienyl) propanenitrile (**2**) : To a solution of 3-acetyloxy-3-(2-thienyl) propanenitrile (5 mmol) in 3 mL of acetone was added phosphate buffer pH = 7.2 and *Pseudomonas cepacia* lipase immobilized on diatomite (PS-D) (500 mg) and incubated at 25°C in an orbital shaker. After 50% completion of the reaction (16 h) as indicated by HPLC, the reaction mixture was filtered and the filtrate was extracted with ethyl acetate and organic solvent was evaporated under reduced pressure to leave a residue. The residue was subjected to column chromatography to separate the unreacted acetate and the alcohol formed. The optical purity of these compounds were determined by HPLC.

Yield of (R)-3-hydroxy-3-(2-thienyl) propanenitrile (***R-*1**) 42%, ee >99%; [α]³⁰_{D} = +32.0 (c 1, CHCl₃);

Yield of (S)-3-acetyloxy-3-(2-thienyl) propanenitrile (***S*-2**) 43%, ee >99%, [α]³⁰_{D} = - 82.1 (c 1, CHCl₃).

### Example 3

Chiral HPLC Analysis: HPLC analysis was performed on an instrument that consisted of a Shimadzu LC-6A system controller, SPD-6A fixed wavelength UV monitor as detector, FCV-100B fraction collector and chromatopac C-R4A data processor as a recording integrator. Analyses were performed by employing chiral column (Chiralcel OJ-H, Daicel) with hexane: isopropanol (85:15) as the mobile phase at a flow rate of 0.75 mL/min and monitored at UV 254 nm. Racemic acetate was prepared by treating its corresponding hydroxy nitrile with acetic anhydride in the presence of pyridine as an authentic sample for comparision on HPLC.

### The main advantages of the present invention are:

Vicinal cyanohydrins or 1,2-cyanohyrins are important and versatile compounds in organic synthesis as these hydroxy nitriles in optically pure form provides a number of opportunities for synthetic manipulations leading to a wide range of chiral synthons like amino alcohols, hydroxy amides, hydroxy esters, hydroxy acids etc., by employing simple methods. Moreover, thiophene can serve as a masked alkyl chain or masked acid group making it more useful intermediate for synthesis chirally pure compounds of biological importance.

In this process chiral 1,2-cyanohydrin has been obtained by lipase mediated resolution of the racemate by transesterification as well as hydrolysis in high enantiomeric excess.

## Claims

1. A chemoenzymatic process for the stereoselective preparation of both *R* and *S* enantiomers of 3-hydroxy-3-(2-thienyl) propanenitrile (**1**), which comprises (i) reacting the cyanohydrin (1) with an acetylating agent in the presence of lipase and then separating the (*R*)-acetate and (*S*)-alcohol obtained ii) enzymatically hydrolysing 3-acetyloxy-3-(2-thienyl)propanenitrile (2) in a phosphate buffer solution to provide (*S*)-acetate and (*R*)-, alcohol.

2. A process as claimed in claim 1 wherein recycling is not required for the products obtained by kinetic resolution since the enantiomeric excess is >99%.

3. A process as claimed in claim 1 wherein the acetylating agent is selected from the group consisting of vinyl acetate or isopropenyl acetate.

4. A process as claimed in claim 1 wherein the acetylation of the cyanohydrin is done in the presence of a lipase in a solvent.

5. A process as claimed in claim 4 wherein the the solvent is selected from the group consisting of diisopropyl ether, toluene, hexane, dioxane, chloroform, t-butylmethyl ether, diethyl ether, tetrahydrofuran, acetonitrile and acetone.

6. A process as claimed in claim 1 wherein the lipase used is selected from the group consisting *of Pseudomonas cepacia* lipase immobilized on modified ceramic particles (PS-C), *Pseudomonas cepacia* lipase immobilized on diatomite (PS-D), *Psedomonas cepacia* (PS), Porcine pancreas lipase (PPL), *Candida cylindracea* lipase (CCL), *Candida rugosa* lipase (CRL) and lipase immobilized from *Mucor miehei* (Lipozyme).

## Patentansprüche

1. Chemoenzymatisches Verfahren zur stereoselektiven Herstellung von sowohl R als auch S Enantiomere von 3-Hydroxy-3-(2-Thienyl)Propannitril (1), das folgendes umfasst: (i) Reagieren des Cyanohydrins (1) mit einem Acetylierungsmittel in der Gegenwart von Lipase und dann Trennen des erhaltenen (R)-Acetats und des erhaltenen (S)-Alkohols (ii) enzymatisches Hydrolysieren von 3-Acetyloxy-3-(2-Thienyl)Propannitril (2) in einer Phosphatpufferlösung, um (S)-Acetat und (R)-Alkohol bereitzustellen.

2. Verfahren gemäß Anspruch 1, worin die Rückführung nicht für die durch kinetische Trennung erhaltenen Produkte erforderlich ist, weil der enantiomere Überschuss >99% ist.

3. Verfahren gemäß Anspruch 1, worin das Acetylierungsmittel aus der Gruppe ausgewählt ist, bestehend aus Vinylacetat oder Isopropenylacetat.

4. Verfahren gemäß Anspruch 1, worin die Acetylierung des Cyanohydrins in der Gegenwart einer Lipase in einer Lösung durchgeführt wird.

5. Verfahren gemäß Anspruch 4, worin die Lösung aus der Gruppe ausgewählt ist, bestehend aus Diisopropylether, Toluol, Hexan, Dioxan, Chloroform; t-Butylmethylether, Diethylether, Tetrahydrofuran, Acetonitril und Aceton.

6. Verfahren gemäß Anspruch 1, worin die verwendete Lipase aus der Gruppe ausgewählt ist, bestehend aus Pseudomonas Cepacia Lipase, immobilisiert auf modifizierten Keramikpartikeln (PS-C), Pseudomonas Cepacia Lipase, immobilisiert auf Diatomit (PS-D), Pseudomonas Cepacia (PS), Schweinepankreas Lipase (PPL), Candida Cylindracea Lipase (CCL), Candida Rugosa Lipase (CRL) und Lipase, immobilisiert von Mucor Miehei (Lipozym).

## Revendications

1. Procédé chimioenzymatique pour la préparation stéréosélective d'énantiomères R et S de 3-hydroxy-3-(2-thiényl)propanenitrile (1), comprenant (i) la mise en réaction de la cyanohydrine (1) avec un agent d'acétylation en présence d'une lipase puis la séparation du (R)-acétate et du (S)-alcool obtenu ii) l'hydrolisation enzymatique du 3-acétyloxy-3-(2-thiényl)propanenitrile (2) dans une solution tampon de phosphate pour fournir du (S)-acétate et du (R)-alcool .

2. Procédé selon la revendication 1, dans lequel le recyclage n'est pas requis pour les produits obtenus par résolution cinétique du fait que l'excès énantiomérique est supérieur à 99%.

3. Procédé selon la revendication 1, dans lequel l'agent d'acétylation est sélectionné dans le groupe consistant en acétate de vinyle ou acétate d'isopropényle.

4. Procédé selon la revendication 1, dans lequel l'acétylation de la cyanohydrine est effectuée en présence d'une lipase dans un solvant.

5. Procédé selon la revendication 4, dans lequel le solvant est sélectionné dans le groupe consistant en éther diisopropylique, toluène, hexane, dioxane, chloroforme, éther t-butylméthylique, éther diéthylique, tétrahydrofuranne, acétonitrile et acétone.

6. Procédé selon la revendication 1, dans lequel la lipase utilisée est sélectionnée dans le groupe consistant en lipase de *Pseudomonas cepacia* immobilisée sur des particules céramiques modifiées (PS-C), lipase de *Pseudomonas cepacia* immobilisée sur diatomite (PS-D), *Pseudomonas cepacia* (PS), lipase du pancréas de porc (PPL), lipase de *Candida cylindracea* (CCL), lipase de *Candida rugosa* (CRL) et lipase immobilisée de *Mucor miehei* (Lipozyme).
